# EUROPEAN PATENT SPECIFICATION

(11) **EP 1 455 831 B1**
(45) Date of publication and mention of the grant of the patent: **31.10.2007**
(21) Application number: 02792102.2
(22) Date of filing: 20.12.2002
(51) Int. Cl.: A61K 45/06, A61K 38/24

(54) **METHOD OF CONTROLLED OVARIAN HYPERSTIMULATION AND PHARMACEUTICAL KIT FOR USE IN SUCH METHOD**
VERFAHREN ZUR REGULIERTEN HYPERSTIMULATION DER EIERSTÖCKE UND PHARMAZEUTISCHES KIT ZUR VERWENDUNG IN DIESEM VERFAHREN
PROCEDE D'HYPERSTIMULATION OVARIENNE CONTROLEE ET TROUSSE PHARMACEUTIQUE A CET USAGE

(30) Priority: 21.12.2001 EP 01205068
(43) Date of publication of application: 15.09.2004
(60) Divisional application: 07116815.7
(73) Proprietor: Pantarhei Bioscience B.V., 3700 AL Zeist (NL)
(72) Inventor: COELINGH BENNINK, Herman, Jan, Tijmen, NL-3971 BE Driebergen (NL); BUNSCHOTEN, Evert, Johannes, NL-5384 EV Heesch (NL)
(74) Representative: van Westenbrugge, Andries
(86) International application number: PCT/NL2002/000853
(87) International publication number: WO 2003/055524

(56) References cited:
- EP-A- 0 417 003
- TANAKA, N. ET AL: "Effects of progesterone and anti-progesterone RU486 on ovarian 3.beta.-hydroxysteroid dehydrogenase activity during ovulation in the gonadotropin-primed immature rat" J. REPROD. FERTIL. (1993), 97(1), 167-72, XP008004092
- GONCALVES, S. CAVACO ET AL: "Influence of an antiprogestin ( onapristone ) on in vivo and in vitro fertilization" ANIM. REPROD. SCI. (1997), 46(1,2), 55-67, XP001086536
- SPITZ, I. M. ET AL: "Progesterone receptor modulators and progesterone antagonists in women's health" STEROIDS (2000), 65(10-11), 807-815, XP004224009
- KRUSCHE, C. A. ET AL: "The progesterone antagonist onapristone retards the advanced endometrial transformation after gonadotropin stimulation in rabbits" STEROIDS (2000), 65(10-11), 773-782, XP001074747
- MARTINEZ, F. ET AL: "Human chorionic gonadotropin and intravaginal natural progesterone are equally effective for luteal phase support in IVF" GYNECOLOGICAL ENDOCRINOLOGY (2000), 14(5), 316-320, XP008004375
- MOCHTAR, M. H. ET AL: "Progesterone alone versus progesterone combined with HCG as luteal suppor in GnRHa/HMG induced IVF cycles: A randomized clinical trial" HUM. REPROD. (1996), 11(8), 1602-1605, XP008004409
- POSACI, CEMAL ET AL: "Progesterone for the luteal support of assisted reproductive technologies clinical options" HUMAN REPRODUCTION (2000), 15(SUPPL. 1), 129-148, XP008004378
- FRIEDLER, S. ET AL: "Luteal support with micronized progesterone following in-vitro fertilization using a down-regulation protocol with gonadotropin-releasin hormone agonist: a comparative study between vaginal and oral administration" HUMAN REPRODUCTION (1999), 14(8), 1944-1948, XP008004379
- MESSINIS, I. E. ET AL: "Effect of mifepristone on folliculogenesis in women treated wit recombinant FSH" CLIN. ENDOCRINOL. (OXFORD) (1997), 46(3), 309-314, XP008004255 cited in the application
- BEIER, HENNING M. ET AL: "Modification of endometrial cell biology using progesterone antagonists t manipulate the implantation window" HUM. REPROD. (1994), 9(SUPPL. 1, PROGESTERONE ANTAGONISTS IN REPRODUCTIVE MEDICINE AND ONCOLOGY), 98-115, XP008004259

## Description

### FIELD OF THE INVENTION

The present invention is concerned with a regimer of controlled ovarian hyperstimulation in mammalian females undergoing in vitro fertilisation (IVF). More particularly the invention relates to a method for treating infertility in mammalian females, which method comprises the administration to said female of a substance having follicle stimulating hormone activity (FSH substance) in an amount effective to stimulate follicular development and of another pharmaceutically active substance to prevent the occurrence of a premature endogenous luteinising hormone (LH) surge, followed by the administration of a meiosis and luteinisation inducing substance (ML substance) having or inducing luteinising hormone (LH) activity in an amount effective to stimulate resumption of meiosis and luteinisation.

Another aspect of the invention is concerned with a pharmaceutical kit for use in the present method of controlled ovarian hyperstimulation.

### BACKGROUND OF THE INVENTION

The ovarian function of mammalian females is regulated by the hypothalamus and pituitary, secreting gonadotropin releasing hormone (GnRH) and gonadotropins respectively. The gonadotropins are follicle stimulating hormone (FSH), which causes follicle maturation, and luteinising hormone (LH), which causes ovulation.

After each menses, the ovaries are stimulated by FSH released by the pituitary to grow a cohort of follicles. These follicles each comprise an oocyte (egg cell) which is enveloped by an orb of granulosa cells. During growth of the follicles several layers of granulosa cells are being formed. Follicle maturation during the normal menstrual cycle occurs in 12-14 days. Gradually, one follicle becomes dominant and the others become atretic. Maturation of the dominant follicle usually takes 5-7 days. As the number of granulosa cells increases more estrogen is secreted by these cells.

Once the dominant follicle has reached maturity, the follicle will burst (ovulate) under the action of a surge of LH which is released by the pituitary in response to the increased blood serum estrogen level (positive feedback). The oocyte is discharged from the follicle into the ampulla of the Fallopian tube, where fertilization may take place. The oocyte or embryo is transported to the uterus in 5-7 days, where implantation may occur in the midluteal phase.

The follicle that has discharged the oocyte is transformed into a new hormone producing organ, the corpus luteum. The corpus luteum produces amongst others progesterone and estrogens. The corpus luteum has a limited lifespan of about 12-14 days, unless pregnancy occurs. During the second part of that period, it ceases functioning, and as a result the blood level of estrogens and progesterone drops. The decline of progesterone causes shedding of the lining of the uterus and thus menstruation.

In particular in the area of ovulation induction, the past decades have shown the development and commercial introduction of numerous drugs assisting in fertility management of infertile couples. Amongst others, these include anti-estrogens (like clomiphene citrate and tamoxifen citrate), pulsatile GnRH, purified and recombinant gonadotropins, and GnRH agonists and antagonists. The specific drugs used and administration regimens chosen largely depend on the goal of the treatment, e.g. the induction of mono-ovulation in anovulatory females or the controlled ovarian hyperstimulation (COH) to induce multiple follicular development as an element in assisted reproductive technologies (ART). Examples of ART methods that are widely used to treat female and/or male factor infertility include intrauterine insemination (IUI) and *in vitro* fertilization (IVF). IVF can be performed with and without intracytoplasmatic sperm injection (ICSI) and includes a subsequent embryo transfer step.

COH is nowadays widely used in ART. First results with COH were disappointing as a result of the occurrence of premature LH surges in at least 30% of the cases. Such a premature LH-surge may incite ovulation of oocytes and may frustrate harvesting of oocytes for *in vitro* fertilisation (IVF). It was found that the introduction of GnRH agonists allowed the prevention of premature LH surges as well as programmation of the treatment cycles. To date GnRH agonists are used in most of the cycles. However, GnRH agonists are peptides, requiring parenteral administration, are expensive and are not devoid of adverse effects (long treatment period, side effects, increased incidence of ovarian hyperstimulation syndrome, etc.).

Recently GnRH antagonists were introduced to prevent premature LH surges, to avoid the side effects related to the use of GnRH agonists and to simplify and shorten treatment protocols. However, there are concerns about the pregnancy rates observed with protocols using GnRH antagonists. Several studies have indicated that pregnancy rates for GnRH antagonists are lower than those achieved with GnRH agonists. Furthermore, like GnRH agonist, GnRH antagonists are peptides requiring parenteral administration, which is less favourable.

An area of key interest to IVF-researchers is the poor implantation rate of IVF embryos, responsible for the relatively low implantation rate per embryo. This has lead to the practice of multiple embryo transfers, which practice in turn has lead to high rates of multiple pregnancies. Such multiple pregnancies are considered a major drawback of ART.

As will be apparent from the above there is a need for a COH-method which does not employ the aforementioned GnRH analogues, but instead uses a substitute which is equally suitable for preventing premature endogenous LH surges, but which produces equal or superior pregnancy rates, can be given orally, gives rise to less side-effects and/or is less expensive.

Messinis et al., "Effect of mifepristone on folliculogenesis in women treated with recombinant FSH", Clinical Endocrinology (1997) 46, 309-314, describe the results of a study of the mechanisms through which mifepristone (RU486) interrupts folliculogenesis. Normally ovulating women undergoing donor intrauterine insemination were investigated during two menstrual cycles treated with gonadotropins. In the first cycle (FSH cycle) the women were given recombinant FSH subcutaneous on days 2, 4, 6, 8 and daily thereafter until the administration of hCG. During the second cycle (FSH + mifepristone cycle) the women received recombinant FSH as above plus mifepristone tablets at what is referred to as a relatively low dose of 50 mg/day from cycle day 2 until the hCG injection. The dose of FSH used did not induce multiple follicular development. Intrauterine insemination was performed only in the FSH cycles.

The results suggest that mifepristone arrests follicular development by delaying the growth of the dominant follicle and that it may prevent the occurrence of LH-surges prior to the hCG injection. It is hypothesised that mifepristone could be used instead of GnRH agonist (or antagonist) during induction of multiple folliculogenesis with FSH for *in vitro* fertilisation to block the LH surge and prevent premature luteinisation. This statement is followed by the remark that it remains to be seen whether mifepristone could also exert blocking effects on the endogenous LH surge during the induction of a greater degree of ovarian stimulation by FSH than that used in the reported study.

Batista et al., "Evidence for a critical role of progesterone in the regulation of the midcycle gonadotropin surge and ovulation", J. Clin Endocrinol Metab (1992), 74, 565-570 report that the antiprogesterone RU486 consistently delayed the timing of the midcycle gonadotropin surge and the subsequent collapse of the dominant follicle. The study was performed in normally cycling women who received 1 mg/day RU486 for 5 or 15 days, starting when the dominant follicle reached 14-16 mm. It is reported that unexpectedly RU486 also delayed the emergence of periovulatory progesterone rise. The addition of progesterone (5-10 mg/day, im, for 2 days) to a 5-day course of RU486 after the emergence of a mature follicle readily induced LH and FSH surges and completely reversed the effects of RU486 at midcycle. It is concluded that RU486 delays the midcycle gonadotropin surge and ovulation by suppressing or antagonising an ovarian progestational signal and that thus progesterone may represent the ultimate ovarian signal to the estrogen-primed hypothalamic-pituitary unit to trigger the gonadotropin surge that leads to ovulation.

Spitz et al. (Steroids (2000), 65(10-11), 807-815 describe the use of anti-progestogen to suppress premature LH-surges. Spitz et al. address the potential use of anti-P in IVF, including its effect on the onset of the LH surge and on endometrial maturation.

Krusche et al. (Steroids (2000), 65 (10-11), 773-782 describe the results of a study in which rabbits received onapriston two day after ovulation induction by means of hCG injection. The authors conclude that post-ovulatory administration of a progesterone antagonist might be a possible strategy to modulate the advanced endometrial development in IVF-cycles.

Fanchin et al., "Effects of vaginal progesterone administration on uterine contractility at the time of embryo transfer", Fertil Steril (2001) 75(6), 1136-1140, report that vaginal progesterone administration starting on the day of oocyte retrieval induced a decrease in uterine contraction frequency on the day of embryo transfer as compared with preovulatory values. Uterine relaxation before embryo transfer is said to likely improve IVF embryo transfer outcome by avoiding displacement of embryos from the uterine cavity.

### SUMMARY OF THE INVENTION

It was found that the aforementioned need for a COH-method that does not employ a GnRH analogue may be met by using an anti-progestogen (anti-P) and a progestogen in a sequential fashion. It is surprising that anti-P can advantageously be used in a method of treating infertility as for some years anti-P has been used clinically for the termination of pregnancy and because it has been proposed to use continuous low doses of anti-P as a novel form of oral contraception.

It was discovered that in a COH-protocol, premature LH-surges may effectively be prevented by administering anti-P during the period when the FSH-stimulated (multiple) follicular development may give rise to such a surge and that high implantation rates may subsequently be achieved by administering a progestogen as soon as anti-P administration is discontinued. Without wishing to be bound by theory, it is believed that anti-P is capable of preventing premature LH-surges by suppressing the ovarian progestational signal that triggers the pituitary to release the LH-surge that leads to ovulation. Because of the relatively high metabolic stability of existing anti-P substances, anti-P will continue to exert its effect for several days after administration is discontinued. The lasting effect of anti-P can adversely affect embryo development (Ghosh et al., "Preimplantation embryo morphology following early luteal phase anti-nidatory treatment with mifepristone (RU486) in the rhesus monkey", J. Hum. Reprod. 2000, 15, 180-188) as well as embryo implantation (Ghosh et al., "Early luteal phase administration of mifepristone inhibits preimplantation embryo development and viability in the rhesus monkey", J. Hum. Reprod., 1997, 12, 575-582). It was found that this adverse effect of anti-P may effectively be counteracted by administering a progestogen, in particular progesterone, around the same time when the ML substance is first administered to stimulate meiosis and luteinisation.

The present COH-method, in comparison to known COH-methods that make use of a GnRH analogue, offers the additional advantage that even if a premature LH-surge occurs (estimated incidence is about 2-3%), negative consequences on subsequent embryo implantation are negated or at least minimised. A premature LH-surge will inevitably trigger early luteinisation, which is associated with rising progesterone levels. A premature increase of the plasma progesterone level is known to have an undesirable impact on the development of the endometrium and particularly on endometrium receptivity. The use of anti-P in accordance with the present invention minimises the impact of a premature increase in plasma progesterone level since the anti-P will effectively block the progesterone receptors or inhibit the endogenous synthesis of progesterone. Thus, with the present method the chance of a successful embryo implantation, in case of a premature LH-surge, is significantly higher than for COH-methods based on GnRH analogues.

In order for ART-methods to be successful, embryo development and endometrial receptivity have to be in synchrony. Some authors have reported that the high hormonal levels associated with COH can lead to advanced endometrium histology. This may induce a shift in the appearance of the implantation window, resulting in an asynchrony between embryo development and endometrium receptivity. Advanced endometrium histology (premature endometrial luteinisation) is believed to be induced by existing methods of COH. The administration of anti-P can prevent the early appearance of the implantation window by suppressing or antagonising the impact of the COH-induced progesterone elevation on endometrium histology. The subsequent administration of an adequate dose of a progestogen counteracts the impeding effect of anti-P on endometrium histology and allows the endometrium to prepare for implantation.

### DETAILED DESCRIPTION OF THE INVENTION

One aspect of the present invention relates to the use of an anti-progestogen (anti-P) in the manufacture of a pharmaceutical composition for use in the treatment of infertility in a mammalian female undergoing in vitro fertilisation (IVF), said method comprising administration to said female of a substance having follicle stimulating hormone activity (FSH substance) selected from the group consisting of urinary FSH (uFSH) and recombinant FSH (recFSH) in an amount effective to stimulate follicular development, preferably multiple follicular development and of anti-P in an effective amount to prevent a premature endogenous LH-surge, followed by the administration of a meiosis and luteinisation inducing substance (ML substance) having or inducing luteinising hormone activity, said ML-substance being selected from the group consisting of recombinant LH (recLH), urinary chorionic gonadotropin (uCG), recombinant CG. orogestogen, gonadotropin releasing hotrmone (GnRH) and mixtures thereof in an amount effective to stimulate resumption of meiosis and luteinisation, and of a progestogen in an amount effective to prevent or suppress symptoms of progesterone antagonism and/or deficiency, wherein the progestogen is administered within 12 hours of the first administration of the ML substance. The phrase "within 12 hours of the first administration" refers to the period starting 12 hours before and ending 12 hours after the first administration.

The term ML substance encompases substances that display a similar functionality as LH, as well as substances which are capable of triggering the pituitary release of LH. Since a progestogen, and in particular progesterone, is capable of triggering the pituitary release of LH, the present invention also encompasses an embodiment wherein, following the administration of FSH and anti-P, progestogen is administered in an amount which is effective to stimulate resumption of meiosis and luteinisation by the triggered release of LH and which amount is also effective to prevent symptoms of progesterone antagonism and/or deficiency.

In a particularly preferred embodiment of the present invention anti-P is administered in an amount equivalent to a daily oral dose of 0.1-42 mg mifepristone (RU486) so as to prevent premature LH-surges. Dosages in excess of 42 mg cause undesirable side effects such as impaired follicular development and a non-receptive endometrium. More preferably, anti-P is administered in an amount equivalent to a daily oral dose of 0.2-22 mg mifepristone, most preferably in an amount equivalent to a daily oral dose of 0.5-12 mg mifepristone.

The multiple follicular development occurring during COH usually produces a particularly strong and premature trigger for the release of LH by the pituitary. Hence, from prior publications such as the aforementioned article by Messinis et al., one would expect that dosages of well in excess of 50 mg would be required to prevent premature LH-surges in such COH-protocols. Unexpectedly, however, it was found that much lower dosages of mifepristone are adequate to prevent LH surges in females undergoing controlled ovarian hyperstimulation.

The present COH-method is employed as part of an IVF-protocol.

The embryo may be transferred into the female uterus during the same cycle in which the COH-protocol is applied and the one or more ova are harvested, but it is also possible to transfer the embryo in a subsequent cycle. Due to the administration of progestogen, however, the present method enables high implantation rates when the COH-protocol and embryo transfer occur within the same cycle.

The term female, whenever referred to in here, relates to mammalian females. Preferably the mammalian female is a homo sapiens. For homo sapiens females are usually biologically capable of child bearing between the age of 12 and 55.

It is crucial that administration of the anti-P is started sufficiently early to minimise the chance of a premature LH-surge. A reliable indicator of the chance of the occurrence of a premature LH-surge is the size of the developing ovarian follicle, and in particular the size of largest of these developing follicles. Preferably, the anti-P is administered at least during the period starting with the moment when the largest developing ovarian follicle has reached an average diameter of 14 mm, preferably of 12 mm, and more preferably of 10 mm, and ending one day prior to the administration of the ML substance in an amount effective to stimulate resumption of meiosis and luteinisation. Usually administration of the anti-P will be continued until the moment the ML substance is administered.

To achieve the desired effect on endometrium histology, the anti-P is administered at least during the period commencing either 6 days after the start of administration of the FSH substance, or at least 4 days prior to the administration of the ML substance in an amount effective to stimulate resumption of meiosis and luteinisation, whichever is the earliest, and ending one day prior to said administration of the ML substance.

The FSH substance is suitably administered at least during the period starting 8 days after the female's spontaneous menses until the day before administration of the ML substance. More preferably the administration of the FSH-substance is commenced no later than 6 days after the female's menses even more preferably on the second day.

It has been demonstrated that COH induces premature disappearance of pinopodes from the endometrium surface. Pinopodes are ultrastructural markers of endometrium receptivity and their appearance is limited to 24 to 48 hours at the time of embryo implantation. The administration of anti-P in accordance with the present method effectively delays the disappearance ofpinopodes and thus prevents a too early implantation window. In a preferred embodiment of the present COH-method, the anti-P is administered in an amount effective to delay the disappearance of pinopodes from the endometrium surface of the female receiving the embryo. Preferably said disappearance is delayed at least until 6 days after the fertilisation.

The administration of anti-P in accordance with the present invention may give rise to lowering of blood serum progesterone levels and/or it may prevent interaction between (endogenous) progesterone and progesterone receptors. Generally 2 types of anti-P substances are distinguished, i.e. receptor antagonists (e.g. mifepristone, lilopristone etc.) and 3β-hydroxy steroid dehydrogenase inhibitors. Receptor antagonists prevent the interaction between progesterone and progesterone receptors, whereas the 3β-hydroxy steroid dehydrogenase inhibitors prevent the endogenous synthesis of progesterone. Both types of anti-P may suitably be employed in the present method. Preferably, however, the anti-P is a receptor antagonist. There is some concern about possible side effects of 3β-hydroxy steroid dehydrogenase inhibitors on e.g. cortisol synthesis. In addition, receptor antagonists offer the advantage that they are effective shortly after administration.

After discontinuation of anti-P administration it may take several days before the original progesterone blood serum levels have been restored to their normal levels and/or before the antagonising effect of anti-P has disappeared. Following the discontinuation of and-P administration, administration of a progestogen in an amount equivalent to a daily intravaginal dosage of 25 to 1000 mg progesterone, is usually sufficient to prevent or suppress symptoms of progesterone antagonism and/or deficiency. Preferably the progestogen is administered in an amount equivalent to a daily intravaginal dosage of 100 to 800 mg progesterone, more preferably to such daily dosage of 300 to 600 mg progesterone. The progestogen and/or the progestogen precursor is suitably administered parenterally (intravaginally, subcutaneously, intramuscularly), orally or intrarectally.

The present invention encompasses the use of a variety of progestogens, including biogenic progestogens (e.g. progesterone), synthetic steroidal progestogens (e.g. levonorgestrel, norgestimate, norethisterone, drospirenone and dydrogesterone) as well as non-steroidal progestogen analogues. Preferably the progestogen employed is a steroidal progestogen. Most preferably the progestogen employed in the present method is progesterone. Progesterone may advantageously be administered intravaginally. In a particularly preferred embodiment progesterone is administered intravaginally at least twice daily.

It was found that the prolonged effects of anti-P after administration of the ML substance may be suppressed very effectively by administering a single, relatively high dose of progestogen within 12 hours of the first administration of the ML substance. This single high dose of progestogen is advantageously administered orally or through injection. Preferably the progestogen is administered in a single dose that is equivalent to an intramuscular dosage of at least 5 mg, more preferably at least 10 mg and most preferably at least 25 mg progesterone.

In a preferred embodiment the progestogen is administered orally or through injection at about the same time (e.g. within 15 minutes) as the ML substance. Particularly preferred is a method wherein progesterone is administered in a single dose by injection or orally, followed by continued intravaginal administration. Naturally the intravaginal administration may commence at the same time as the injection or oral administration of progesterone. The single dose of progesterone can be administered effectively by injection, particularly intramuscular injection. In case the progesterone is also employed as the ML substance, dosages need to be higher than in case it is solely administered to negate the effect of the anti-P. The maximum level of progesterone that is administered in a single dose usually does not exceed 600 mg. Preferably the maximum dosage does not exceed 400 mg, more preferably it does not exceed 200 mg.

The use of antiprogestogen in accordance with the present invention may have a prolonged effect on blood serum progesterone levels and/or the interaction of endogenous progesterone with the progesterone receptors. Hence it is preferred to apply an administration regimen wherein the progestogen is administered in at least 2 doses at intervals of at least 8 hours within a period of 3 weeks, preferably within a period of 2 weeks, more preferably within 1 week.

It is crucial that the progestogen is administered at around the same time when the administration of the ML substance commences, i.e. within 12 hours after the first administration of the ML substance. If not, implantation rates will be adversely affected because of the unfavourable effect of the anti-P on the development of the endometrium during this phase of the IVF-protocol. Consequently, in the present method, the administration of the progestogen and/or the progestogen precursor is always started prior to the harvesting of the one or more ova. Preferably the progestogen is administered within 1 hour of the first administration of the ML substance. The present method employs a ML substance to stimulate meiosis and luteinisation after the lead follicles have reached maturity and administration of FSH and anti-P is discontinued. The objective of administering the ML substance at this stage of the cycle is to mimic the LH surge which occurs during the normal menstruation cycle and which induces ovulation, resumption of meiosis and luteinisation. The amount of ML substance administered in accordance with the present method preferably is equivalent to a subcutaneous dose of at least 2,000 I.U. urinary chorionic human gonadotropin (uhCG), more preferably to a subcutaneous dose of 5,000-10,000 I.U. uhCG. Preferably the ML substance is administered in a single oral or parenteral dose. Most preferably the ML substance is administered subcutaneously or orally.

It is well known that both FSH and LH may be isolated from female urine. LH isolated from urine is less suitable for use in the present method as it has a very short *in vivo* half-life (t_{1/2}: 10-20 minutes) and is metabolised very quickly. LH obtained from a recombinant cell line (recLH) is much more stable (t_{1/2}: 12-13 hours). Consequently, in a particularly preferred embodiment, if LH is used to prevent or suppress symptoms of LH deficiency, said LH is obtained from a recombinant cell line. The high dose of the ML substance used to stimulate resumption of meiosis and luteinisation is preferably recLH or uhCG, most preferably recLH.

The FSH substance used in the present method is selected from the group consisting of urinary FSH (uFSH) and recombinant FSH (recFSH). Although FSH of urinary origin performs almost equally well as recFSH, it is noted that the isolation of active principles from bodily fluids is associated with the risk of transfer of diseases. Hence, in a preferred embodiment, the FSH substance is FSH obtained from a recombinant cell line.

Throughout this document, the term "parenteral administration" encompasses all modes of administration, requiring injection, implantation or topical administration, except for the oral/intestinal route. Suitable examples of parenteral administration include intramuscular, intravenous, subcutaneous, intravaginal, transdermal and intranasal administration.

In a preferred embodiment of the present method, anti-P is administered parenterally or orally in an amount that is equivalent to a daily oral dosage of 5-400 µg mifepristone per kg bodyweight, more preferably of 10-150 µg mifepristone per kg bodyweight. More preferably anti-P is administered subcutaneously or orally. Most preferably anti-P is administered orally.

The FSH substance may suitably be administered parenterally or orally, preferably in an amount that is equivalent to a daily subcutaneous dosage of 1 to 15 I.U. recFSH per kg bodyweight. Most preferably the FSH substance is administered subcutaneously. As mentioned herein before, the present method can suitably employ any anti-progestogens known in the art, including both receptor antagonists and 3β-hydroxy steroid dehydrogenase inhibitors. An extensive list of examples of suitable anti-P substances can be found in WO 01/47490. Preferably the anti-P is selected from the group consisting of mifepristone (RU486), lilopristone (ZK98734), onapristone (ZK98299), CDB-2914 (HRP2000), Org31710, Org31167, Org31343, Org33628, ZK137316, ZK230211, gestrinone(R-2323), ORF9371, RTI3021-012, RTI3021-020, RTI3021-022, epostane, azastene, trilostane, cyanoketone, precursors of these anti-P substances and mixtures these anti-P substances and/or precursors. More preferably the anti-P is selected from the group consisting of mifepristone, lilopristone, anopristone, precursors of these substances and mixtures thereof.

As mentioned herein before the present COH-method offers the advantage that it does not require the use of a GnRH agonist or antagonist. However, it is feasible to employ anti-P in combination with a relatively low dose of antagonist (e.g. between 2 and 20 µg ganirelix per kg bodyweight) so as to reduce the drawbacks associated with the use of such GnRH analogues. Consequently the combined use of anti-P and GnRH analogues is encompassed by the present invention

Best results are obtained with the present COH-method if the FSH substance and the anti-P are administered at least once daily. Preferably also the progestogen is administered at least once daily.

As mentioned herein before the present method also encompasses an embodiment wherein following the administration of FSH and anti-P, progestogen is administered in an amount which is effective to prevent symptoms of progesterone antagonism and/or deficiency and which moreover facilitates the endogenous release of LH from the pituitary gland in an amount effective to stimulate resumption of meiosis and luteinisation. In other words, in such an embodiment of the present invention the progestogen is employed as the ML substance. This is a particularly preferred embodiment of the present invention as it enables a COH protocol that requires only 3 different pharmacological substances, i.e. FSH substance, anti-P and progestogen.

Another aspect of the present invention relates to a pharmaceutical kit for use in a method of controlled ovarian hyperstimulation in mammalian females undergoing IVF, said kit comprising a parenteral dosage unit containing a FSH substance, selected from the group consisting of a FSH and recFSH a parenteral or oral dosage unit containing an anti-P and a parenteral or oral dosage unit containing progesterone. More preferably the kit comprises a parenteral, even more preferably an intravaginal dosage unit containing progesterone and/or a precursor thereof. The intravaginal dosage unit may suitably take the form of a vaginal gel, a vaginal capsule or a vaginal ring.

Preferably the kit also comprises a parenteral dosage unit containing an ML substance. Preferably the kit comprises one or two dosage units containing an ML substance. Most preferably the kit comprises one dosage unit containing an ML substance. Preferably the pharmaceutical kit according to the invention comprises the FSH substance in an amount which is equivalent to a subcutaneous dose of between 50 and 1000 IU recFSH, the ML substance in an amount equivalent to a subcutaneous dosage of between 2,000 and 20,000 uhCG, anti-P in an amount equivalent to an oral dosage of between 0.1 and 600 mg mifepristone and a progestogen in an amount equivalent to an intravaginal dosage of between 10 and 1000 mg progesterone.

As mentioned herein before it is advantageous to administer a single high dose of progesterone through injection at about the same time as the first administration of the ML substance. Hence, the present kit preferably comprises a single parenteral dosage unit for administration by injection. Preferably said dosage unit contains at least 5 mg, more preferably at least 10 mg and most preferably at least 25 mg of progesterone. Generally the dosage unit will contain progesterone in an amount of less than 600 mg, preferably of less than 400 mg and most preferably of less than 200 mg. The parenteral progesterone dosage unit is preferably suited for intramuscular or subcutaneous administration. In a particularly preferred embodiment of the present invention, the kit comprises a single parenteral dosage unit for administration by injection, which dosage unit contains at least 5 mg progesterone, and an intravaginal dosage unit which contains between 10 and 1000 mg progesterone.

The parenteral dosage units within the present kit are preferably cartridges for subcutaneous self-administration, containing a sterile liquid formulation.

### EXAMPLES

### Example 1

An open-label, uncontrolled clinical trial is performed to investigate the efficacy, safety, and tolerability of premature LH-surge prevention in 30 female subjects undergoing COH and subsequent IVF and embryo transfer (ET), using a daily oral dosage of 40 mg mifepristone from day 6 of recombinant FSH treatment up to and including the day of hCG treatment and thrice daily intravaginal progesterone supplementation with 200 mg starting immediately after the hCG treatment for a period of up to 12 weeks.

Although this treatment is suitable for all types of IVF patients (e.g. within the age range 18 to 45 years, with or without displaying polycystic ovarian syndrome and with or without a regular cycle), the following selection criteria are set forth in the investigation: healthy female partners of infertile couples; age at the time of screening between 20 and 39 years; a body mass index (BMI) between 19 and 29 kg/m²; a regular menstrual cycle, and willing to give a written informed consent.

Prior to the first administration of recombinant FSH a blood sample is taken for hormone analysis (estradiol and LH) and a standard urinary hCG assay is performed to exclude pregnancy. Recombinant FSH treatment is started at day 2 or 3 of menses onwards by a daily subcutaneous injection until the day of hCG administration. During the first 5 days of recombinant FSH treatment the dose is fixed at 150 IU. Starting at day 6, blood samples for hormone analysis are taken every two days prior to drug administration and ultrasonographic monitoring of follicle growth is performed to adjust, if necessary, the daily injectable dose of recombinant FSH.

A subcutaneous injection with urinary hCG (5.000 IU) and an intramuscular injection of 50 mg progesterone are administered simultaneously, when at least three follicles exceed a diameter of 17 mm as measured by ultrasound scan and subsequently oocyte retrieval is performed 30-38 hours later.

A daily oral administration of 40 mg mifepristone is found to be efficacious in preventing premature LH-rises (above levels of 10 IU/L) from day 6 of recombinant FSH treatment until the day of hCG treatment. In addition, daily oral administration of 40 mg mifepristone is well tolerated and shows no adverse effects.

### Example 2

Oocytes are retrieved from a human female undergoing COH using the procedure as set forth in example 1, the oocytes are subsequently fertilized *in vitro* and two days later no more than two embryos are transferred to the uterus of the patient, resulting in a vital pregnancy as assessed by ultrasound scan.

### Example 3

Using the procedure as set forth in example 1, with the proviso that, instead of using a daily dose of 40 mg mifepristone, mifepristone is used at a daily dose of 20 mg, a similar clinical outcome is obtained at a lower anti-P exposure level in the female subjects. A daily oral administration of 20 mg mifepristone is found to be efficacious in preventing premature LH-rises (above levels of 10 IU/L) from day 6 of recombinant FSH treatment until the day of hCG treatment. In addition, daily oral administration of 20 mg mifepristone is well tolerated and shows no adverse effects.

### Example 4

Oocytes are retrieved from a human female undergoing COH using the procedure as set forth in example 3, the oocytes are subsequently fertilized *in vitro* and two days later no more than two embryos are transferred to the uterus of the patient, resulting in a vital pregnancy as assessed by ultrasound scan.

### Example 5

Using the procedure as set forth in example 1, with the proviso that, instead of using a daily dose of 40 mg mifepristone, mifepristone is used at a daily dose of 10 mg, a similar clinical outcome is obtained at a lower anti-P exposure level in the female subjects. A daily oral administration of 10 mg mifepristone is found to be efficacious in preventing premature LH-rises (above levels of 10 IU/L) from day 6 of recombinant FSH treatment until the day of hCG treatment. In addition, daily oral administration of 10 mg mifepristone is well tolerated and shows no adverse effects.

### Example 6

Oocytes are retrieved from a human female undergoing COH using the procedure as set forth in example 5, the oocytes are subsequently fertilized *in vitro* and two days later maximally two embryos are transferred to the uterus of the patient, resulting in a vital pregnancy as assessed by ultrasound scan.

### Example 7

An open-label, randomized and controlled clinical study is performed in oocyte donors undergoing COH and in whom premature LH-surges are prevented by either standard GnRH agonist treatment (5 oocyte donors) or mifepristone treatment (5 oocyte donors) with the objective to compare endometrial receptivity.

In women treated with 40 mg orally administered mifepristone, the procedure as set forth in example 1 is used, with the proviso that instead of administering progesterone for 12 weeks, daily intravaginal progesterone supplementation with 400 mg is started immediately after the hCG treatment and continued until the day of endometrial biopsy (day 7 after hCG injection in donors).

To oocyte donors in the reference group, undergoing COH and receiving GnRH agonist, treatment with GnRH agonist is started in the midluteal phase of the preceding menstrual cycle and given concomitantly with recombinant FSH from day 2 or 3 of the COH cycle up to and including the day of hCG treatment. Vaginal progesterone supplementation is started in the GnRH-treated oocyte donors directly after oocyte retrieval (36-38 hours after hCG injection) and continued until the day of endometrial biopsy (day 7 after hCG injection).

Endometrial samples are taken from all oocyte donors. Endometrial tissues are embedded and sections are processed by routine hematoxylin and eosin staining for endometrial glandular dating by standard histological criteria and are processed for scanning electron microscopy to examine the presence of pinopodes.

In biopsies from oocyte donors undergoing mifepristone treatment, typically in-phase glandular dating is observed. In contrast, the majority of endometrial biopsies from oocyte donors using GnRH agonist treatment are showing advanced dating. Furthermore, in endometrial biopsies prepared for scanning electron microscopy, pinopodes are readily visible in mifepristone treated oocyte donors, whereas only a minority of samples from GnRH-treated oocyte donors show the presence of pinopodes.

### Example 8

An open-label, uncontrolled clinical investigation is performed in 5 healthy female volunteers undergoing COH and subsequent IVF and ET, using the procedure as set forth in example 1, with the proviso that in stead of administering hCG a single intramuscular injection of 50 mg progesterone is administered to allow an endogenous LH surge to occur, inducing resumption of meiosis and luteinisation. A single intramuscular injection of 50 mg progesterone is found to be efficacious in allowing endogenous LH-surges to occur. This is evidenced by the rise of serum LH concentration and the retrieval of good quality oocytes when harvested at 36-3836 hours after starting progesterone treatment.

### Example 9

Oocytes are retrieved from a human female undergoing COH using the procedure as set forth in example 8, the oocytes are subsequently fertilized *in vitro* and two days later no more than two embryos are transferred to the uterus of the patient, resulting in a vital pregnancy as assessed by ultrasound scan.

## Claims

1. Use of an anti-progestogen (anti-P) in the manufacture of a pharmaceutical composition for use in the treatment of infertility in a mammalian female undergoing in vitro fertilisation (IVF), comprising administration to said female of:
■ a substance having follicle stimulating hormone activity (FSH substance) selected from the group consisting of urinary FSH (uFSH) and; recombinant FSH (recFSH) in an amount effective to stimulate follicular development; and
■ anti-P in an effective amount to prevent a premature endogenous LH-surge;
followed by administration of:
■ a meiosis and luteinisation inducing substance (ML substance) having or inducing luteinising hormone activity, said ML substance being selected from the group consisting of recombinant LH (recLH), urinary chorionic gonadotropin (uCG), recombinant CG, progestogen, gonadotropin releasing hormone (GnRH) and mixtures thereof, in an amount effective to stimulate resumption of meiosis and luteinisation; and
■ a progestogen in an amount effective to prevent or suppress symptoms of progesterone antagonism and/or deficiency, wherein the progestogen is administered within 12 hours of the first administration of the ML substance.

2. Use according to claim 1, wherein the anti-P is administered in an amount equivalent to a daily oral dose of 0.1-42 mg, preferably of 0.2-22 mg mifepristone, more preferably of 0.5-12 mg mifepristone.

3. Use according to claim 1 or 2, wherein the progestogen is administered in an amount equivalent to a daily intravaginal dosage of 25 to 1000 mg progesterone, more preferably 300-600 mg progesterone.

4. Use according to any one of claims 1-3, wherein the infertility treatment is employed as part of an IVF protocol.

5. Use according to claim 4, wherein the IVF protocol is carried out within one cycle.

6. Use according to any one of claims 1-5, wherein the anti-P is administered at least during the period starting with the moment when the largest developing ovarian follicle has reached an average diameter of 14 mm, preferably of 12 mm, more preferably of 10 mm, and ending one day prior to the administration of the ML substance in an amount effective to stimulate resumption of meiosis and luteinisation.

7. Use according to any one of claims 1-6, wherein the anti-P is administered at least during the period commencing either 6 days after the start of administration of the FSH substance, or at least 4 days prior to the administration of the ML substance in an amount effective to stimulate resumption of meiosis and luteinisation, whichever is the earliest, and ending one day prior to said administration of the ML substance.

8. Use according to any one of claims 1-7, wherein the FSH substance is administered at least during the period starting 8 days, preferably 6 days and more preferably 2 days after the female's spontaneous menses until the day before administration of the ML substance in an amount effective to stimulate resumption of meiosis and luteinisation.

9. Use according to any one of claims 4-8, wherein the anti-P is administered in an amount effective to delay the disappearance of pinopodes from the endometrium surface of the female at least until 6 days after the fertilisation.

10. Use according to any one of claims 1-9, wherein the anti-P is administered parenterally or orally, preferably in an amount equivalent to a daily oral dosage of 5 µg to 400 µg mifepristone per kg bodyweight.

11. Use according to any one of claims 1-10, wherein the anti-P is selected from the group consisting of mifepristone (RU486), lilopristone (ZK98734), onapristone (ZK98299), CDB-2914 (HRP2000), Org31710, Org31167, Org31343, Org33628, ZK137316, ZK230211, gestrinone(R-2323), ORF9371, RTI3021-012, RTI3021-020, RTI3021-022, epostane, azastene, trilostane, cyanoketone.

12. Use according to any one of claims 1-11, wherein the progestogen is administered within 1 hour of the first administration of the ML substance.

13. A pharmaceutical kit for use in controlled ovarian hyperstimulation in mammalian females undergoing in vitro fertilisation, said kit comprising a parenteral or oral dosage unit containing a FSH substance selected from the group consisting of urinary FSH (uFSH)-, and recombinant FSH (recFSH), a parenteral or oral dosage unit containing an anti-P and a single dosage unit for administration by injection containing progesterone.

14. A pharmaceutical kit according to claim 14 comprising the FSH substance in an amount which is equivalent to a subcutaneous dose of between 50 and 1000 I.U. recFSH, anti-P in an amount equivalent to an oral dosage of between 0.1 and 600 mg mifepristone and progesterone in an amount equivalent to an intravaginal dosage of between 10 and 1000 mg progesterone.

## Patentansprüche

1. Verwendung eines Anti-Progestogens (Anti-P) bei der Herstellung einer pharmazeutischen Zusammensetzung zur Verwendung bei der Behandlung von Unfruchtbarkeit bei einem weiblichen Säugetier, das sich einer In-vitro-Fertilisation (IVF, künstliche Befruchtung) unterzieht, wobei die Verwendung die Verabreichung von Folgendem an das weibliche Säugetier umfasst:
• eine Substanz, die follikelstimulierende Hormonaktivität hat (FSH-Substanz) und aus der Gruppe ausgewählt ist, die aus einem Urin-FSH (uFSH) und rekombinantem FSH (recFSH) besteht, in einer Menge, die wirksam ist, um Follikelentwicklung zu stimulieren; und
• Anti-P in einer wirksamen Menge, um einen vorzeitigen endogenen LH-Stoß zu verhindern;
gefolgt von der Verabreichung von Folgendem:
• einer Meiose und Luteinisierung induzierenden Substanz (ML-Substanz), die Luteinisierungshormonaktivität hat oder induziert, wobei die ML-Substanz aus der Gruppe ausgewählt ist, die besteht aus: rekombinantem LH (recLH), Urin-Chorion-Gonadotropin (uCG), rekombinantem CG, Progestogen, Gonadotropin-freisetzendes-Hormon (GnRH) und Gemischen davon, in einer Menge, die wirksam ist, um die Wiederaufnahme von Meiose und Luteinisierung zu stimulieren; und
• ein Progestogen in einer Menge, die wirksam ist, um Symptome von Progesteron-Gegenwirkung und/oder -Mangel zu verhindern oder zu unterdrücken, wobei das Progestogen innerhalb von 12 h nach der ersten Verabreichung der ML-Substanz verabreicht wird.

2. Verwendung nach Anspruch 1, wobei das Anti-P in einer Menge verabreicht wird, die einer oralen Tagesdosis von 0,1-42 mg, bevorzugt von 0,2-22 mg Mifepriston, stärker bevorzugt von 0,5-12 mg Mifepriston äquivalent ist.

3. Verwendung nach Anspruch 1 oder 2, wobei das Progestogen in einer Menge verabreicht wird, die einer intravaginalen Tagesdosis von 25 bis 1000 mg Progesteron, stärker bevorzugt 300-600 mg Progesteron äquivalent ist.

4. Verwendung nach einem der Ansprüche 1-3, wobei die Unfruchtbarkeitsbehandlung als Teil eines IVF-Protokolls angewandt wird.

5. Verwendung nach Anspruch 4, wobei das IVF-Protokoll in einem Zyklus ausgeführt wird.

6. Verwendung nach einem der Ansprüche 1-5, wobei das Anti-P mindestens während des Zeitraums verabreicht wird, der zu dem Zeitpunkt beginnt, zu dem das größte sich entwickelnde Ovarialfollikel einen mittleren Durchmesser von 14 mm, bevorzugt von 12 mm, stärker bevorzugt von 10 mm erreicht hat, und einen Tag vor der Verabreichung der ML-Substanz in einer Menge, die wirksam ist, um die Wiederaufnahme von Meiose und Luteinisierung zu stimulieren, endet.

7. Verwendung nach einem der Ansprüche 1-6, wobei das Anti-P mindestens während des Zeitraums verabreicht wird, der entweder 6 Tage nach dem Beginn der Verabreichung der FSH-Substanz oder mindestens 4 Tage vor der Verabreichung der ML-Substanz in einer Menge, die wirksam ist, um die Wiederaufnahme von Meiose und Luteinisierung zu stimulieren, beginnt, je nachdem, welcher Zeitpunkt der frühere ist, und einen Tag vor der Verabreichung der ML-Substanz endet.

8. Verwendung nach einem der Ansprüche 1-7, wobei die FSH-Substanz mindestens während des Zeitraums verabreicht wird, der 8 Tage, bevorzugt 6 Tage und stärker bevorzugt 2 Tage nach der Spontanmenstruation des weiblichen Säugetiers beginnt, bis zu dem Tag vor der Verabreichung der ML-Substanz in einer Menge, die wirksam ist, um die Wiederaufnahme von Meiose und Luteinisierung zu stimulieren.

9. Verwendung nach einem der Ansprüche 4-8, wobei das Anti-P in einer Menge verabreicht wird, die wirksam ist, um das Verschwinden von Pinopodien von der Endometriumoberfläche des weiblichen Säugetiers um mindestens 6 Tage nach der Fertilisation zu verzögern.

10. Verwendung nach einem der Ansprüche 1-9, wobei das Anti-P parenteral oder oral, bevorzugt in einer Menge verabreicht wird, die einer oralen Tagesdosis von 5 µg bis 400 µg Mifepriston je kg Körpergewicht äquivalent ist.

11. Verwendung nach einem der Ansprüche 1-10, wobei das Anti-P aus der Gruppe ausgewählt ist, die besteht aus: Mifepriston (RU486), Lilopriston (ZK98734), Onapriston (ZK98299), CDB-2914 (HRP2000), Org31710, Org31167, Org31343, Org33628, ZK137316, ZK230211, Gestrinon(R-2323), ORF9371, RTI3021-012, RTI3021-020, RTI3021-022, Epostan, Azasten, Trilostan, Cyanoketon.

12. Verwendung nach einem der Ansprüche 1-11, wobei das Progestogen innerhalb 1 Stunde nach der ersten Verabreichung der ML-Substanz verabreicht wird.

13. Pharmazeutisches Set zur Verwendung bei der gesteuerten Ovarialhyperstimulation in weiblichen Säugetieren, die sich einer In-Vitro-Fertilisation unterziehen, wobei das Set Folgendes aufweist: eine parenterale oder orale Dosiseinheit, die eine FSH-Substanz enthält, die aus der Gruppe ausgewählt ist, die aus Urin-FSH (uFSH) und rekombinantem FSH (recFSH) besteht, eine parenterale oder orale Dosiseinheit, die ein Anti-P enthält, und eine Einzeldosiseinheit, die Progesteron enthält, zur Verabreichung durch Injektion.

14. Pharmazeutisches Set nach Anspruch 14, das Folgendes aufweist: die FSH-Substanz in einer Menge, die einer subkutanen Dosis zwischen 50 und 1000 IU recFSH äquivalent ist, Anti-P in einer Menge, die einer oralen Dosis zwischen 0,1 und 600 mg Mifepriston äquivalent ist, und Progesteron in einer Menge, die einer intravaginalen Dosis zwischen 10 und 1000 mg Progesteron äquivalent ist.

## Revendications

1. Utilisation d'un anti-progestogène (anti-P) dans la fabrication d'une composition pharmaceutique destinée à être employée dans le traitement de la stérilité chez un mammifère femelle subissant une fécondation in vitro (FIV), comprenant l'administration à ladite femelle :
d'une substance présentant une activité d'hormone de stimulation folliculaire (substance FSH), choisie dans le groupe constitué de la FSH urinaire (uFSH) et de la FSH recombinante (recFSH), en une quantité efficace pour stimuler le développement folliculaire ; et
d'un anti-P en une quantité efficace pour prévenir une montée de LH endogène prématurée ;
suivie de l'administration :
d'une substance induisant une méiose et une lutéinisation (substance ML) présentant ou induisant une activité d'hormone lutéinisante, ladite substance ML étant choisie dans le groupe constitué de la LH recombinante (recLH), la gonadotrophine chorionique urinaire (uCG), la CG recombinante, le progestogène, l'hormone de libération de la gonadotrophine (GnRH) et des mélanges de ceux-ci, en une quantité efficace pour stimuler la reprise de la méiose et de la lutéinisation ; et
d'un progestogène en une quantité efficace pour prévenir ou réprimer les symptômes d'antagonisme de la progestérone et/ou de carence en progestérone, le progestogène étant administré dans les 12 heures suivant la première administration de la substance ML.

2. Utilisation selon la revendication 1, dans laquelle l'anti-P est administré en une quantité équivalente à une dose orale quotidienne de 0,1 à 42 mg de mifépristone, de préférence de 0,2 à 22 mg de mifépristone, de manière davantage préférée de 0,5 à 12 mg de mifépristone.

3. Utilisation selon la revendication 1 ou 2, dans laquelle le progestogène est administré en une quantité équivalente à une posologie intravaginale quotidienne de 25 à 1 000 mg de progestérone, de manière davantage préférée de 300 à 600 mg de progestérone.

4. Utilisation selon l'une quelconque des revendications 1 à 3, dans laquelle le traitement de la stérilité est employé en tant que partie intégrante d'un protocole FIV.

5. Utilisation selon la revendication 4, dans laquelle le protocole FIV est effectué au cours d'un cycle.

6. Utilisation selon l'une quelconque des revendications 1 à 5, dans laquelle l'anti-P est administré au moins durant la période commençant par le moment où le plus grand follicule ovarien en développement a atteint un diamètre moyen de 14 mm, de préférence de 12 mm, plus préféremment de 10 mm, et s'achevant un jour avant l'administration de la substance ML en une quantité efficace pour stimuler la reprise de la méiose et de la lutéinisation.

7. Utilisation selon l'une quelconque des revendications 1 à 6, dans laquelle l'anti-P est administré au moins durant la période commençant soit 6 jours après le début de l'administration de la substance FSH, soit au moins 4 jours avant l'administration de la substance ML en une quantité efficace pour stimuler la reprise de la méiose et de la lutéinisation, quelle que soit la première des deux, et s'achevant un jour avant ladite administration de la substance ML.

8. Utilisation selon l'une quelconque des revendications 1 à 7, dans laquelle la substance FSH est administrée au moins durant la période commençant 8 jours, de préférence 6 jours, et de manière davantage préférée 2 jours, après les menstruations spontanées de la femelle jusqu'au jour précédant l'administration de la substance ML en une quantité efficace pour stimuler la reprise de la méiose et de la lutéinisation.

9. Utilisation selon l'une quelconque des revendications 4 à 8, dans laquelle l'anti-P est administré en une quantité efficace pour retarder la disparition des pinopodes de la surface de l'endomètre de la femelle au moins jusqu'à 6 jours après la fécondation.

10. Utilisation selon l'une quelconque des revendications 1 à 9, dans laquelle l'anti-P est administré par voie parentérale ou orale, de préférence en une quantité équivalente à une posologie orale quotidienne de 5 µg à 400 µg de mifépristone par kg de poids du corps.

11. Utilisation selon l'une quelconque des revendications 1 à 10, dans laquelle l'anti-P est choisi dans le groupe constitué de mifépristone (RU486), lilopristone (ZK98734), onapristone (ZK98299), CDB-2914 (HRP2000), Org31710, Org31167, Org31343, Org33628, ZK137316, ZK230211, gestrinone (R-2323), ORF9371, RTI3021-012, RTI3021-020, RTI3021-022, épostane, azastène, trilostane, cyanocétone.

12. Utilisation selon l'une quelconque des revendications 1 à 11, dans laquelle le progestogène est administré dans un délai d'une heure après la première administration de la substance ML.

13. Trousse pharmaceutique destinée à être utilisée dans une hyperstimulation ovarienne contrôlée chez des femelles mammifères subissant une fécondation in vitro, ladite trousse comprenant une unité posologique parentérale ou orale contenant une substance FSH choisie dans le groupe constitué de la FSH urinaire (uFSH) et de la FSH recombinante (recFSH), une unité posologique parentérale ou orale contenant un anti-P et une unité posologique unique pour administration par injection contenant de la progestérone.

14. Trousse pharmaceutique selon la revendication 14, comprenant la substance FSH en une quantité qui est équivalente à une dose sous-cutanée comprise entre 50 et 1 000 I.U. de recFSH, l'anti-P en une quantité qui est équivalente à une posologie orale comprise entre 0,1 et 600 mg de mifépristone, et la progestérone en une quantité qui est équivalente à une posologie intravaginale comprise entre 10 et 1 000 mg de progestérone.
